# EUROPEAN PATENT SPECIFICATION

(11) **EP 2 315 853 B1**
(45) Date of publication and mention of the grant of the patent: **12.06.2013**
(21) Application number: 09808435.3
(22) Date of filing: 31.07.2009
(51) Int. Cl.: C12Q 1/68

(54) **METHOD FOR DETECTING AND IDENTIFYING CANDIDA SPECIES**
MEDIUM FÜR DEN NACHWEIS UND DIE IDENTIFIKATION VON CANDIDA-SPEZIES
PROCÉDÉ DE DÉTECTION ET D'IDENTIFICATION D'ESPÈCES DE CANDIDA

(30) Priority: 18.08.2008 MY 0803143
(43) Date of publication of application: 04.05.2011
(73) Proprietor: Universiti Putra Malaysia, Selangor Darul Ehsan (MY)
(72) Inventor: SEOW, Heng Fong, 58100 Kuala Lumpur (MY); LESLIE, Than Thian Lung, 93350 Kuching Sarawak (MY); CHONG, Pei Pei, Subang Jaya 47610 Selangor (MY)
(74) Representative: Katzameyer, Michael
(86) International application number: PCT/MY2009/000109
(87) International publication number: WO 2010/021534

(56) References cited:
- WO-A1-2007/038578
- WO-A2-2006/123295
- US-A1- 2008 102 449
- DATABASE GENBANK 01 January 2004 XP008142541 Database accession no. AY207074
- DATABASE GENBANK 30 October 2005 XP008142540 Database accession no. DQ227972
- DATABASE GENBANK 01 January 2004 XP008142551 Database accession no. AY207068
- DATABASE GENBANK 14 July 1998 XP008142542 Database accession no. AF022719
- DATABASE GENBANK 04 April 2001 XP008142548 Database accession no. AJ401692
- DATABASE GENBANK 31 October 2007 XP008142545 Database accession no. EF065173
- DATABASE GENBANK 11 February 1998 XP008142549 Database accession no. CLU70503
- DATABASE GENBANK 01 January 2004 XP008142546 Database accession no. AY207079
- DATABASE GENBANK 11 February 1998 XP008142580 Database accession no. CRU70506
- DATABASE GENBANK 01 January 2004 XP008142552 Database accession no. AY207080
- LUO, G. ET AL.: 'Rapid Identification of Pathogenic Fungi Directly from Cultures by Using Multiplex PCR' JOURNAL OF CLINICAL MICROBIOLOGY vol. 40, no. 8, 2002, pages 2860 - 2865, XP008128796
- CHEN, Y.C. ET AL.: 'Identification of Medically Important Yeasts Using PCR-Based Detection of DNA Sequence Polymorphisms in the Internal Transcribed Spacer 2 Region of the rRNA Genes' JOURNAL OF CLINICAL MICROBIOLOGY vol. 38, no. 6, 2000, pages 2302 - 2310, XP008142544

## Description

### FIELD OF INVENTION

The present invention relates to design and use of amplification oligonucleotides used for the detection and identification of *Candida* species and a rapid detection and identification method therefor. In more particular, the present invention relates to custom-designed primer sequences which are applicable for the detection and identification of ten *Candida* species and a rapid semi-nested polymerase chain reaction (PCR) method for detecting and identifying the same.

### BACKGROUND OF THE INVENTION

*Candida* is a genus of yeast which includes a wide range of species that are usually endosymbionts or commensals. However, the genus *Candida* does include a significant numbers of species that are pathogenic and cause candidiasis or thrush in humans and other animals, especially in the immunocompromised patients. *Candida* species is currently the fourth highest contributor to nosocomial bloodstream infections in the United States.

One of the many types of diseases that are caused by this genus is invasive candidiasis. Invasive candidiasis is a fungal infection that occurs when *Candida* species enter the blood, causing bloodstream and then spreading throughout the body. It is also known as "disseminated candidiasis", "systemic candidiasis" or "hematogenous candidiasis". It has been reported that the use of antineoplastic and immunosuppressive agents, broad-spectrum antibiotics, prosthetics devices, grafts and aggressive surgeries have caused the incidence of these cases of infection to increase. In addition, factors such as patients with bums, neutropenia, HIV infection and pancreatitis also contribute to the uptrend of the cases.

There are many species in the genus *Candida* that cause invasive candidiasis, namely *C. albicans, C. tropicalis, C. glabrata, C. parapsilosis* and *C*. *krusei.* Each of these species has different susceptibility patterns towards antifungal drugs and some are even resistance towards them thus it is imperative to distinguish them to avoid wrong choices of antifungals administration that leads to unwanted side effects.

Besides the efforts of searching for more potent antifungals against these yeasts, diagnosis has always played its role in reducing unnecessary side effects, hospital expenditures and deaths. Until today, the gold standards for fungal diagnosis are culture and microscopical methods. Conventional detection method to detect *Candida* from blood is through blood culture and it was reported to have sensitivity of 58%. Serological methods are also one of the means in diagnosing invasive candidiasis. Many publications on these methods with varying sensitivity and specificity have been reported. Nevertheless, there is a trend in publications stating that combination of different methods from conventional to the most-up-to-date methods can in fact improve the outcome of diagnostic results. With the advent of molecular detections, diagnoses of invasive candidiasis have improved quite significantly.

There are some patented technologies over the prior arts relating to detection and identification method of *Candida* spp. Most of these methods relate to methods for detecting only the most common species of *Candida* which is C. *albicans.*

U.S. Patent No. US5910409 discloses methods and reagents for detecting fungal pathogens in a biological sample. This invention provides materials and methods for sensitively and selectively screening biological samples for the presence of C. *albicans.* This invention discloses nucleic acids, reagents and primers for DNA amplification. The amplification specifically targets a 526 base pairs nucleotide sequence from DNA containing C. *albicans.*

Another detection method of *C*. *albicans* is embodied by U.S. Patent No. US5545525. According to this invention, a DNA probe is adapted to be used as PCR target for the detection. The presence of the organism is determined by amplicon detection on electrophoresis gel or hybridization. However, this probe and the method invented are designed to target the 25S rRNA of *C*. *albicans.* It also detects trace amount of *C*. *stellatoidea.*

Of interest in connection with methods and compositions for detecting and identifying species of *Candida* is U.S. Patent No. US2008102449. The composition invented are combinations of oligonucleotides including pairs of forward and reverse primers for polymerase chain reactions (PCR), wherein each primer pair is capable of priming the synthesis of an amplicon specific to only one of the species. This invention also discloses probes capable of detecting these amplicons and sequencing primers to determine the nucleotide sequence in the amplicons. Another embodiment of this invention is methods to isolate nucleic acid, amplify the nucleotides, probe to ascertain the presence of the amplicons and determine the nucleotides sequence. However, the methods disclosed can be used to specifically identify only four species, including *C*. *albicans, C. glabrata, C. parapsilosis* and *C*. *tropicalis.*

There is another PCR identification of four medically important *Candida* spp. using one primer pair and four species-specific probes embodied in U.S. Patent No. US5426026. In this invention, the DNA oligonucleotides can amplify and distinguish *C*. *albicans, C*. *glabrata, C*. *parapsilosis* and *C*. *tropicalis.* However, the identification method disclosed is only based on comparison of the length of the amplicons. The species-specific probes are generated by the PCR amplified products.

Another U.S. Patent No. US6017699 also discloses an identification and quantification process of important *Candida* species by PCR. This invention relates to a set of DNA primers which, when utilized in conjunction with the PCR, can amplify and differentiate DNA from five medically important *Candida* species. In another embodiment of this invention, the PCR amplified products generated by the primers can also be used to create specific probes which can also detect and confirm the five species of *Candida.* The method allows for early diagnosis and treatment of an infection as this is a technique for quantitatively determining the amount of *Candida* in a sample and selecting an appropriate dosage or type of antifungal agent for treating that infection caused by *C*. *albicans, C. parapsilosis, C. tropicalis, C. glabrata* and *C*. *krusei.*

Still another invention relating to the detection of *C*. *albicans* is disclosed in Canadian Patent No. CA2608742. This invention uses DNA fragment isolated from the internal transcribed spacer-1 (ITS 1) and internal transcribed spacer-2 (ITS 2) regions of *Candida.* The molecular method is based on the PCR in a multiplex variant in order to detect and identify a wider range of *Candida* species, utilizing the existence of conserved or variable sequences in fungal ribosomal genes and internal primers specifically for each one of the *Candida.*

The methods disclosed by the patented technologies include a wide range of molecular techniques yet there are only a few types of *Candida* species specifically identified. Clearly there is a need for the present invention to develop a more innovative method for detecting and identifying clinically important *Candida* spp. which is more rapid and user-friendly than all patented technologies so as to ensure an earlier diagnosis of the infection and treatment.

### SUMMARY OF INVENTION

The primary object of the present invention is to develop sets of amplification oligonucleotides or primers for species-specifically detecting and identifying species of *Candida.*

Another object of the present invention is to provide a diagnostic kit having primer sets for the detection and identification of ten species of clinically important *Candida.*

Still another object of the present invention is a method for species-specifically detecting and identifying ten species of clinically important *Candida,* including *C*. *albicans, C. dubliniensis, C. glabrata, C. guilliermondii, C. kefyr, C. krusei, C. lusitaniae, C. parapsilosis, C. rugosa* and *C*. *tropicalis.*

The present invention also aims to provide a rapid semi-nested PCR method for species-specifically detecting and identifying *Candida* species using custom-designed amplification oligonucleotides or primers targeting the ITS 1 and ITS 2 of the Candida species.

At least one of the preceding objects is met, in whole or in part, by the present invention, in which one of the embodiments of the present invention describes an isolated oligonucleotide comprising a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10, or any of complementary sequences thereof selected from the group consisting of SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22.

Another embodiment of the present invention is a primer for polymerase chain reaction (PCR)-based amplification comprising an amplification nucleotide sequences selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10, or any of complementary sequences thereof selected from the group consisting of SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22.

Still another embodiment of the present invention is a polymerase chain reaction (PCR)-based amplification kit for detecting and identifying *Candida* species in a biological sample comprising at least one of primers having nucleotide sequences selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10, or any of complementary sequences thereof selected from the group consisting of SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22.

In a further embodiment, the present invention discloses a method for detecting and identifying *Candida* species comprising the step of amplifying conserved DNA segment of *Candida* species in a biological sample using a primer set having a nucleotide sequence of SEQ ID NO: 11 and a nucleotide sequence of SEQ ID NO: 12, and contacting the amplified conserved DNA segment with a primer set having a nucleotide sequence of SEQ ID NO: 11 and a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10.

One of the preferred embodiments of the present invention discloses a method for species-specifically detecting and identifying *Candida albicans* by contacting the amplified conserved DNA segment with a primer set having a nucleotide sequence of SEQ ID NO: 11 and a nucleotide sequence of SEQ ID NO: 1 or the complementary sequence thereof.

Another preferred embodiment of the present invention discloses a method for species-specifically detecting and identifying *Candida dubliniensis* by contacting the amplified conserved DNA segment with a primer set having a nucleotide sequence of SEQ ID NO: 11 and a nucleotide sequence of SEQ ID NO: 2 or the complementary sequence thereof.

Yet another preferred embodiment of the present invention discloses a method for species-specifically detecting and identifying *Candida glabrata* by contacting the amplified conserved DNA segment with a primer set having a nucleotide sequence of SEQ ID NO: 11 and a nucleotide sequence of SEQ ID NO: 3 or the complementary sequence thereof.

Still another preferred embodiment of the present invention is a method for species-specifically detecting and identifying *Candida guilliermondii* by contacting the amplified conserved DNA segment with a primer set having a nucleotide sequence of SEQ ID NO: 11 and a nucleotide sequence of SEQ ID NO: 4 or the complementary sequence thereof.

The present invention also discloses a method for species-specifically detecting and identifying *Candida kefyr* by contacting the amplified conserved DNA segment with a primer set having a nucleotide sequence of SEQ ID NO: 11 and a nucleotide sequence of SEQ ID NO: 5 or the complementary sequence thereof.

Another preferred embodiment of the present invention is a method for species-specifically detecting and identifying *Candida krusei* by contacting the amplified conserved DNA segment with a primer set having a nucleotide sequence of SEQ ID NO: 11 and a nucleotide sequence of SEQ ID NO: 6 or the complementary sequence thereof.

Preferably, the *Candida lusitaniae* can be species-specifically detected and identified by contacting the amplified conserved DNA segment with a primer set having a nucleotide sequence of SEQ ID NO: 11 and a nucleotide sequence of SEQ ID NO: 7 or the complementary sequence thereof.

Preferably, the *Candida parapsilosis* can be species-specifically detected and identified by contacting the amplified conserved DNA segment with a primer set having a nucleotide sequence of SEQ ID NO: 11 and a nucleotide sequence of SEQ ID NO: 8 or the complementary sequence thereof.

Preferably, the *Candida rugosa* can be species-specifically detected and identified by contacting the amplified conserved DNA segment with a primer set having a nucleotide sequence of SEQ ID NO: 11 and a nucleotide sequence of SEQ ID NO: 9 or the complementary sequence thereof.

Preferably, the *Candida tropicalis* can be species-specifically detected and identified by contacting the amplified conserved DNA segment with a primer set having a nucleotide sequence of SEQ ID NO: 11 and a nucleotide sequence of SEQ ID NO: 10 or the complementary sequence thereof.

The amplification oligonucleotides invented is useful for detecting the presence of *Candida* nucleic acids in test samples from human such as blood, blood derived samples, tissues, tissue-derived samples, other body fluids and body samples.

In the present invention, semi-nested PCR method is applied by which the sensitivity of the assay can be increased as semi-nested PCR eliminates the occurrence of false positive and dilutes any inhibitors found in the sample during the first round PCR.

One skilled in the art will readily appreciate that the present invention is well adapted to carry out the objects and obtain the ends and advantages mentioned, as well as those inherent therein. The embodiments described herein are not intended as limitations on the scope of the invention.

### BRIEF DESCRIPTION OF THE DRAWINGS

For the purpose of facilitating an understanding of the invention, there is illustrated in the accompanying drawing the preferred embodiments from an inspection of which when considered in connection with the following description, the invention, its construction and operation and many of its advantages would be readily understood and appreciated.

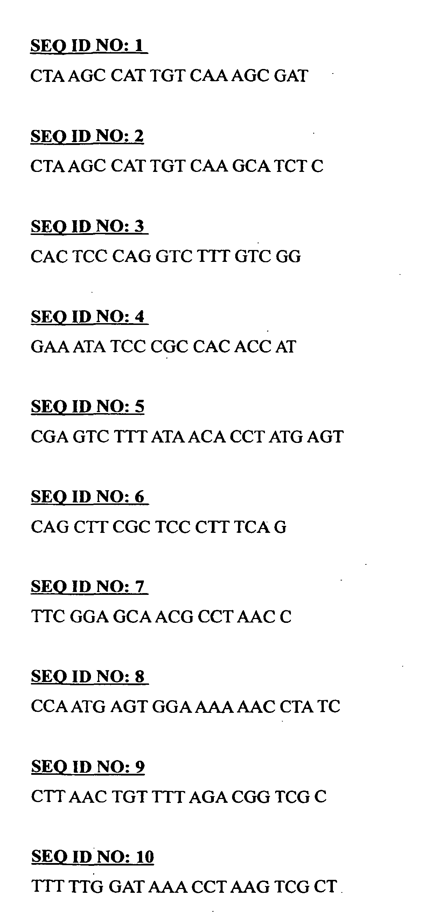

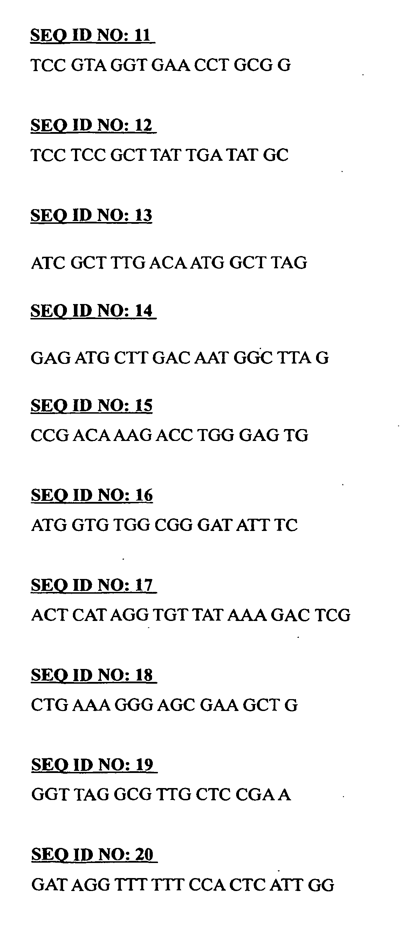

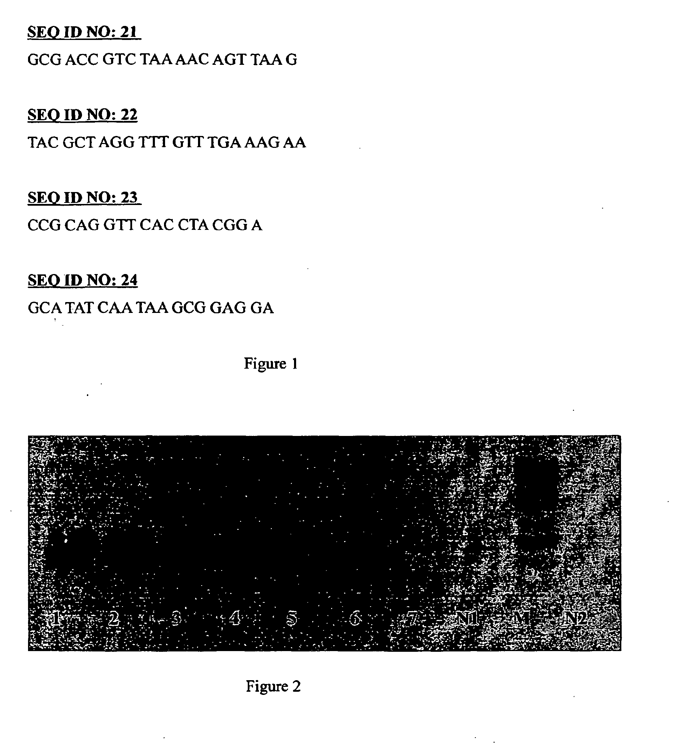

- **Figure 1**: is the nucleotide sequence of the oligonucleotides used for the detection and identification of then *Candida* species as described by the preferred embodiment of the present invention.
- **Figure 2**: shows the results of sensitivity test for semi-nested PCR detection of *C*. *albicans.* Lanes: 1, 10⁶ cells; 2, 10⁵ cells; 3, 10⁴ cells; 4, 10³ cells; 5, 10² cells; 6, 10¹ cells; 7, 10° cell; N1, no template control first round PCR; M, 100bp DNA ladder marker; and N2, no template control second round PCR.

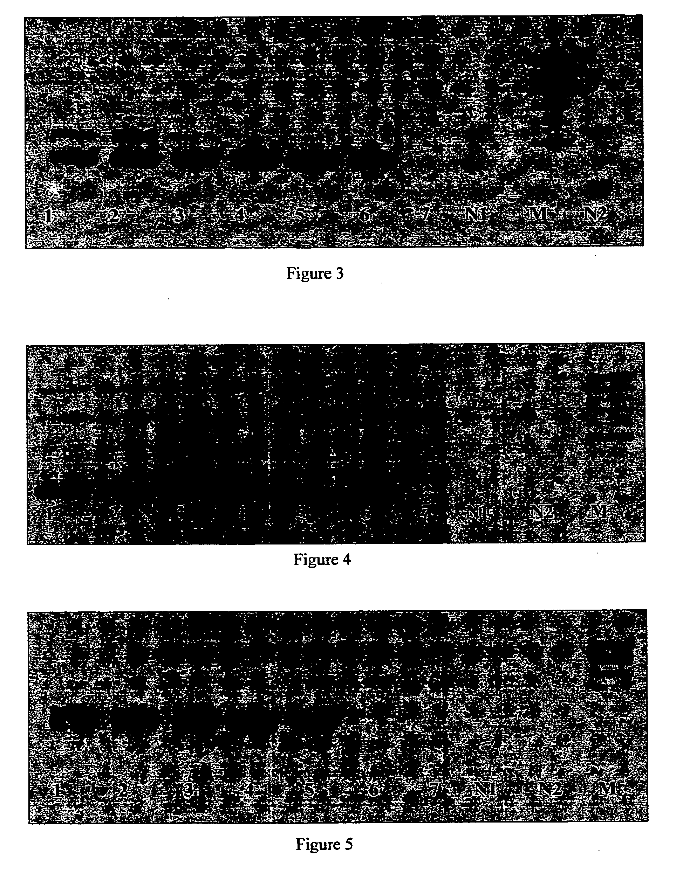

- **Figure 3**: shows the results of sensitivity test for the semi-nested PCR detection of *C*. *dubliniensis.* Lanes: 1, 10⁶ cells; 2, 10⁵ cells; 3, 10⁴ cells; 4, 10³ cells; 5, 10² cells; 6, 10¹ cells; 7, 10⁰ cell; N1, no template control first round PCR; M, 100 bp DNA ladder marker; and N2, no template control second round PCR.
- **Figure 4**: shows the results of sensitivity test for the semi-nested PCR detection of *C*. *glabrata.* Lanes: 1, 10⁶ cells; 2, 10⁵ cells; 3, 10⁴ cells; 4, 10³ cells; 5, 10² cells; 6, 10¹ cells; 7, 10⁰ cell; N1, no template control first round PCR; N2, no template control second round PCR and M, 100 bp DNA ladder marker.
- **Figure 5**: shows the results of sensitivity test for the semi-nested PCR detection of *C*. *guilliermondii.* Lanes: 1, 10⁶ cells; 2, 10⁵ cells; 3, 10⁴ cells; 4, 10³ cells; 5, 10² cells; 6, 10' cells; 7, 10⁰ cell; N1, no template control first round PCR; N2, no template control second round PCR and M, 100 bp DNA ladder marker.

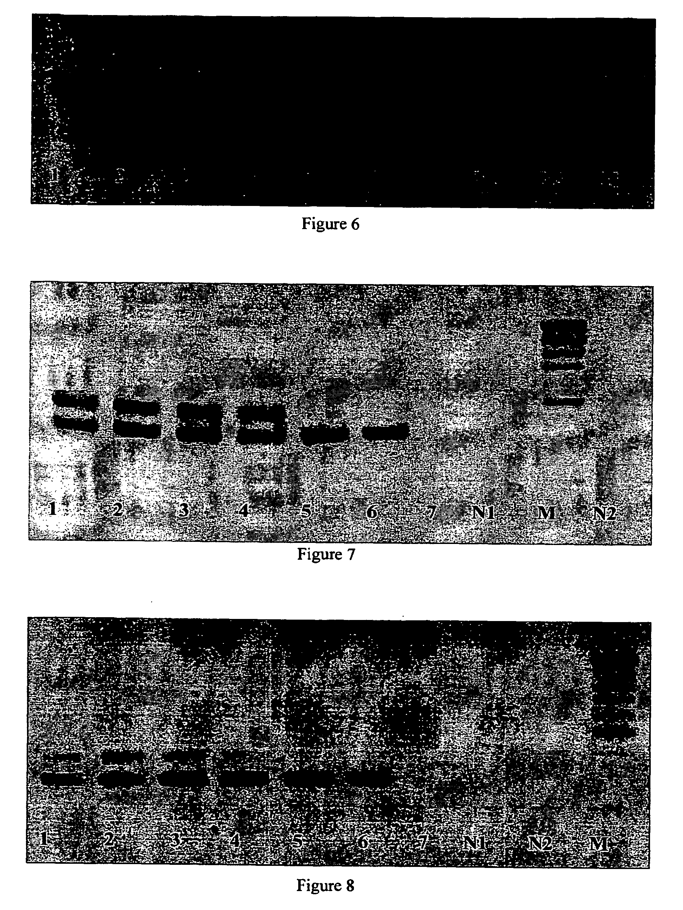

- **Figure 6**: shows the results of sensitivity test for the semi-nested PCR detection of *C*. *kefyr.* Lanes: 1, 10⁶ cells; 2, 10⁵ cells; 3, 10⁴ cells; 4, 10³ cells; 5, 10² cells; 6, 10' cells; 7, 10° cell; N1, no template control first round PCR; N2, no template control second round PCR and M, 100 bp DNA ladder marker.
- **Figure 7**: shows the results of sensitivity test for the semi-nested PCR detection of *C*. *krusei.* Lanes: 1, 10⁶ cells; 2, 10⁵ cells; 3, 10⁴ cells; 4, 10³ cells; 5, 10² cells; 6, 10' cells; 7, 10° cell; N1, no template control first round PCR; M, 100 bp DNA ladder marker; and N2, no template control second round PCR.
- **Figure 8**: shows the results of sensitivity test for the semi-nested PCR detection of *C*. *lusitaniae.* Lanes: 1, 10⁶ cells; 2, 10⁵ cells; 3, 10⁴ cells; 4, 10³ cells; 5, 10² cells; 6, 10¹ cells; 7, 10° cell; N1, no template control first round PCR; N2, no template control second round PCR and M, 100 bp DNA ladder marker.

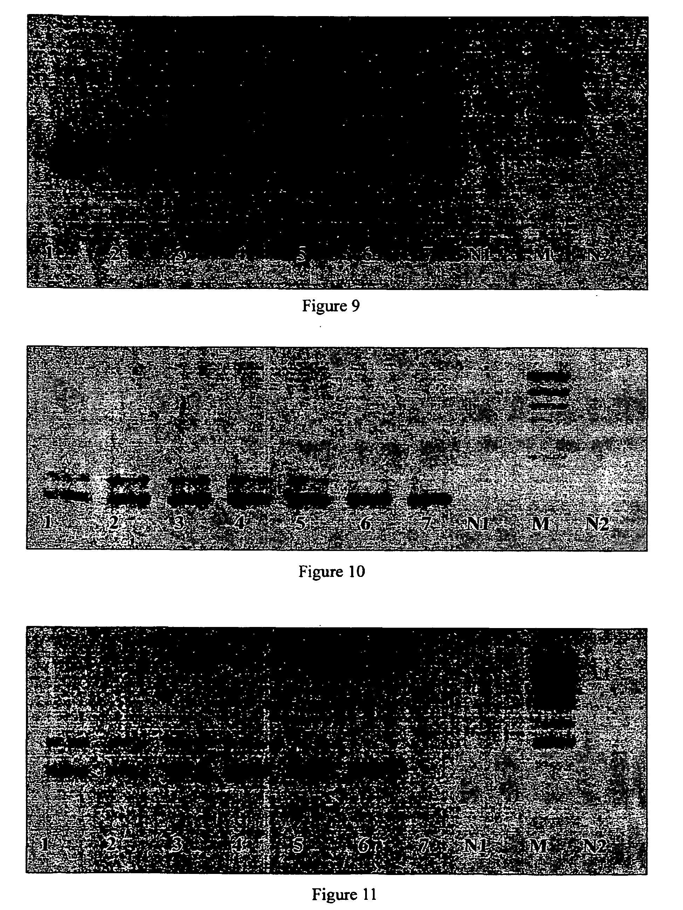

- **Figure 9**: shows the results of sensitivity test for the semi-nested PCR detection of *C*. *parapsilosis.* Lanes: 1, 10⁶ cells; 2, 10⁵ cells; 3, 10⁴ cells; 4, 10³ cells; 5, 10² cells; 6, 10' cells; 7, 10° cell; N1, no template control first round PCR; M, 100 bp DNA ladder marker; and N2, no template control second round PCR.
- **Figure 10**: shows the results of sensitivity test for the semi-nested PCR detection of *C*. *rugosa.* Lanes: 1, 10⁶ cells; 2, 10⁵ cells; 3, 10⁴ cells; 4, 10³ cells; 5, 10² cells; 6, 10¹ cells; 7, 10° cell; N1, no template control first round PCR; M, 100 bp DNA ladder marker; and N2, no template control second round PCR.
- **Figure 11**: shows the results of sensitivity test for the semi-nested PCR detection of *C*. *tropicalis.* Lanes: 1, 10⁶ cells; 2, 10⁵ cells; 3, 10⁴ cells; 4, 10³ cells; 5, 10² cells; 6, 10¹ cells; 7, 10° cell; N1, no template control first round PCR; M, 100 bp DNA ladder marker; and N2, no template control second round PCR.

### DETAILED DESCRIPTION OF THE INVENTION

The present invention relates to design and use of amplification oligonucleotides used for the detection and identification of *Candida* species and a rapid detection and identification method therefor. In more particular, the present invention relates to custom-designed primer sequences which are applicable for the detection and identification of ten *Candida* species and a rapid semi-nested polymerase chain reaction (PCR) method for detecting and identifying the same.

Hereinafter, the invention shall be described according to the preferred embodiments of the present invention and by referring to the accompanying description and drawings. However, it is to be understood that limiting the description to the preferred embodiments of the invention and to the drawings is merely to facilitate discussion of the present invention and it is envisioned that those skilled in the art may devise various modifications without departing from the scope of the appended claim.

The following terms used throughout the specification have the indicated meanings unless expressly indicated to have a different meaning.

By "target nucleic acid" is meant a nucleic acid having a target nucleotide sequence.

By "oligonucleotide" is meant a single-stranded nucleotide polymer made of more than two nucleotide subunits covalently joined together. Preferably, the number of nucleotide units present is ranged from 10 to 100, most preferably it is ranged from 12 to 50. The sugar group of the nucleotide subunits is deoxyribose. The nucleotide subunits of an oligonucleotide may be joined by phosphodiester linkages, phosphorothioate linkages, methyl phosphonate linkages or by other rare or non-naturally-occurring linkages that do not prevent amplification of the oligonucleotide. Furthermore, an oligonucleotide may have uncommon nucleotides or non-nucleotide moieties. An oligonucleotide as defined herein is a nucleic acid, DNA. Amplification oligonucleotides of a defined sequence may be produced by techniques known to those of ordinary skill in the art, such as by chemical synthesis. As intended by this disclosure, an oligonucleotide does not consist of wild-type chromosomal DNA or the *in vivo* transcription products thereof.

By "target nucleic acid sequence", "target nucleotide sequence" or "target sequence" is meant a specific deoxyribonucleotide sequence comprising all or a part of the nucleotide sequence of a single-stranded nucleic acid molecule, and the deoxyribonucleotide sequence complementary thereto.

By "complementary" is meant that the nucleotide sequences of similar regions of two single-stranded nucleic acids, or to different regions of the same single-stranded nucleic acid have a nucleotide base composition that allow the single strands to bind together in a stable double-stranded hydrogen-bonded region under stringent amplification conditions. When a contiguous sequence of nucleotides of one single-stranded region is able to form a series of hydrogen-bonded base pairs with an analogous sequence of nucleotides of the other single-stranded region, such that A is paired with T and C is paired with G, the nucleotides sequences are complementary.

By "amplification oligonucleotide" is meant an oligonucleotide capable of binding to a target nucleic acid sequence and acting as a primer for nucleic acid synthesis for the initiation of nucleic acid synthesis. An amplification oligonucleotide may or may not have a 3' terminus which is modified to prevent or lessen the amount of primer extension. An amplification oligonucleotide as defined herein will preferably be 10 to 50 nucleotides in length; most preferably 15 to 25 nucleotides in length. While the amplification oligonucleotides of the present invention may be chemically synthesized and such oligonucleotides are not naturally-occurring nucleic acids.

By "nucleic acid amplification" or "target amplification" is meant increasing the number of nucleic acid molecules having at least one target nucleic acid sequence.

By "antisense" or "negative sense" is meant having a nucleic sequence complementary to that of a reference nucleic acid sequence.

By "sense", "same-sense" or "positive sense" is meant having a nucleic acid sequence analogous to that of a reference nucleic acid sequence.

"Phylogenetically closely related" means that the organisms are closely related to each other in an evolutionary sense and therefore would have a higher total nucleic acid sequence homology than organisms that are more distantly related. Organisms occupying adjacent and next to adjacent positions on the phylogenetic tree are closely related. Organisms occupying positions further away than adjacent or next to adjacent positions on the phylogenetic tree will still be closely related if they have significant total nucleic acid sequence homology.

The present invention discloses an isolated oligonucleotide comprising a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10, or any of complementary sequences thereof selected from the group consisting of SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22.

The amplification oligonucleotides invented is useful for detecting the presence of *Candida* nucleic acids in biological test samples from human such as blood, blood derived samples, tissues, tissue-derived samples, other body fluids and body samples.

Another embodiment of the present invention is a primer for polymerase chain reaction (PCR)-based amplification comprising an amplification nucleotide sequences selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10, or any of complementary sequences thereof selected from the group consisting of SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22. These primers, when contacted with a nucleic acid polymerase under amplification conditions, will bind to or cause extension through a nucleic acid region having their complimentary sequences.

The useful amplification oligonucleotides are designed to be complementary to specific regions of the ribosomal RNA (rRNA) or ribosomal DNA (rDNA) nucleotide sequences of *Candida,* or oligonucleotides having a nucleic acid sequence substantially corresponding to a specific portion from species of *Candida* rRNA or rDNA nucleotide sequences, comprising SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 and its complementary sequences, comprising SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 and SEQ ID NO: 22.

As these amplification oligonucleotides are derived from the rRNA of pathogenic *Candida,* a superior detection assay is obtained due to the multicopy nature of the gene. The amplification oligonucleotides function by amplifying target *Candida* ITS1 and ITS2 rRNA and/or rDNA gene sequences under amplification conditions. In preferred embodiments, the amplification oligonucleotides described herein, when used together, can distinguish *Candida* spp. from other microorganisms found in clinical samples such as blood or tissues as well as from among the species in the genus. Accordingly, the amplification oligonucleotides may be used in an assay to specifically detect and/or amplify *Candida* species-derived nucleic acids.

Another aspect of the present invention includes compositions for detecting *Candida* species which are nucleic acid hybrids formed between an oligonucleotide of the present invention and a specific region of a nucleotide polymer from *Candida* species. Generally, the nucleotide polymer contains a nucleic acid sequence that substantially corresponds to an oligonucleotide sequence of the present invention or its complement and is derived from the rDNA encoding the ribosomal RNA of the *Candida* species. The oligonucleotides present in these compositions are amplification oligonucleotide. Thus, compositions of the present invention contain one or more amplification oligonucleotides.

Nucleotides compositions of the present invention containing an oligonucleotide primer amplifying its target sequence are useful for creating an initiation site for a polymerase at the 3' end of the primer, and/or providing a template for extension of the 3' end of the target sequence.

The present invention also contemplates diagnostic systems in kit form. A diagnostic system of the present invention may include a kit which contains, in an amount sufficient for at least one assay, amplification primers of the present invention in a packaging material. Typically, the kits would also include instructions for use of the packaged primers.

The PCR-based amplification kit for detecting and identifying *Candida* species in a biological sample is described in another embodiment of the present invention. This kit comprises at least one of primers having nucleotide sequences selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10, or any of complementary sequences thereof selected from the group consisting of SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 22.

The PCR kit contains one or more of the oligonucleotides of the present invention including amplification oligonucleotides or primer sets. According to the preferred embodiment of the present invention, a kit of the present invention includes at least one amplification oligonucleotide capable of specifically distinguishing *C*. *albicans, C. dubliniensis, C. glabrata, C. guilliermondii, C. kefyr, C. krusei, C. lusitaniae, C. parapsilosis, C. rugosa* or *C*. *tropicalis* from other microorganisms.

The various components of the diagnostic kit may be provided in various forms. For example, the required enzymes, the nucleotide triphosphates, the primers may be provided as a lyophilized reagent. These lyophilized reagents may be premixed before lyophilization so as to form a complete mixture with the proper ratio of each of the components ready for use in the assay. In addition, the diagnostic systems of the present invention may contain a reconstitution reagent for reconstituting the lyophilized reagents of the kit.

According to the preferred embodiment of the present invention, the enzymes, nucleotides, triphosphates and required cofactors for the enzymes are provided as a single lyophilized reagent that when reconstituted forms a proper reagent for use in the present methods. In these preferred kits, a lyophilized primer agent may also be provided. Typical packaging materials would include solid matrices such as glass, plastic, paper, foil, micro particles and the like, capable of holding within fixed limits amplification primer of the present invention. Thus, for example, a package made from packaging materials can be a glass vial used to contain sub-milligram (i.e. picogram, nanogram etc.) quantities of a contemplated primer or it could be a microtiter plate well to which the primers of the present invention have been operatively affixed, i.e., linked so as to be capable of participating in a detection method of the present invention.

In a further embodiment, the present invention discloses a method for detecting and identifying *Candida* species comprising the step of amplifying conserved DNA segment of *Candida* species in a biological sample using a primer set having a nucleotide sequence of SEQ ID NO: 11 and a nucleotide sequence of SEQ ID NO: 12, and contacting the amplified conserved DNA segment with a primer set having a nucleotide sequence of SEQ ID NO: 11 and a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10.

The nucleotide sequence of SEQ ID NO: 11 and a nucleotide sequence of SEQ ID NO: 12 embodied herein can be used as the universal sequences for all fungi as they are designed to target the conserved DNA segment. Therefore, this amplification oligonucleotides or primer set can be used to amplify the conserved DNA segment of *Candida* spp. All the oligonucleotides or primer sets used in the present invention are commercially obtained.

Semi-nested PCR method is applied by which the sensitivity of the assay can be increased as this method eliminates the occurrence of false positive and dilutes any inhibitors found in the sample during the first round PCR. Therefore, a second round of PCR is carried out by using the amplified DNA fragment obtained from the first round of PCR which is primed by the nucleotide sequence of SEQ ID NO: 11 and SEQ ID NO: 12.

Further, the present invention contemplates methods for detecting the presence of *Candida* species by contacting a test sample under optimized amplification condition with nucleic acid primers capable of preferentially amplifying under optimized amplification assay conditions to *Candida* species target nucleic acid. However, one skilled in the art will understand that the exact assay conditions, primers used will vary depending on the particular assay format used and the source of the sample.

One of the preferred embodiments of the present invention discloses a method for species-specifically detecting and identifying *Candida albicans* by contacting the amplified conserved DNA segment with a primer set having a nucleotide sequence of SEQ ID NO: 11 and a nucleotide sequence of SEQ ID NO: 1 or the complementary sequence thereof.

Detection and identification of *Candida dubliniensis* can be performed by substituting the SEQ ID NO: 1 with SEQ ID NO: 2. Likewise, the amplification of *C*. *glabrata, C. guilliermondii, C. kefyr, C. krusei, C. lusitaniae, C. parapsilosis, C. rugosa* and *C*. *tropicalis* can be carried out by substituting the SEQ ID NO: 1 with SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO: 10, respectively.

As set forth in the foregoing description, the amplification oligonucleotides or primer sets of the present invention are directed to particular *Candida* ITS1 and ITS2 regions. The amplification oligonucleotides described and claimed herein comprise two sets of amplification oligonucleotides. Members of the set of amplification oligonucleotides are able to bind with a nucleic acid having or substantially corresponding to one of the following nucleotide sequences, which are SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO: 10.

In preferred embodiments, these amplification oligonucleotides have or substantially correspond to these complementary sequences, including SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 and SEQ ID NO: 22.

A wide variety of methods are available to detect an amplified target sequence. For example, the nucleotide substrates or the primers can include a detectable label which is incorporated into newly synthesized DNA. The resulting labelled amplification product is then separated from the unused labelled nucleotides or primers and the label is detected in the separated product fraction.

Substances which can serve as useful detectable labels are well known in the art and include radioactive isotopes, fluorescent compounds, chemiluminescent compounds, chromophores, as well as ligands such as biotin and haptens which, while not directly detectable, can be readily detected by a reaction with labeled forms of their specific binding partners, e.g. avidin and antibodies, respectively.

The present disclosure includes as contained in the appended claims, as well as that of the foregoing description. Although this invention has been described in its preferred form with a degree of particularity, it is understood that the present disclosure of the preferred form has been made only by way of example and that numerous changes in the details of construction and the combination and arrangements of parts may be resorted to without departing from the scope of the invention.

### EXAMPLE

Examples are provided below to illustrate different aspects and embodiments of the present invention. These examples are not intended in any way to limit the disclosed invention, which is limited only by the claims.

Amplification oligonucleotides or primers specific for *Candida albicans* were designed using sequences determined in prospective target areas using primers complementary to the 18S rDNA of *Candida albicans* (ATCC NO. 14053), *Candida dubliniensis* (ATCC NO. MYA-178), *Candida glabrata* (ATCC No. 2001), *Candida guilliermondii* (ATCC No. 6260), *Candida kefyr* (ATCC NO. 66028), *Candida krusei* (ATCC NO. 6258), *Candida lusitaniae* (ATCC NO. 66035), *Candida parapsilosis* (ATCC NO. 22019), *Candida rugosa* (ATCC NO. 10571), *Candida tropicalis* (ATCC NO. 750), *Aspergillus flavus* (ATCC NO. 10124), *Aspergillus fumigatus* (ATCC NO. 36607), *Aspergillus lentulus* (ATCC NO. MYA-3566), *Aspergillus nidulans* (ATCC NO. 10074), *Aspergillus niger* (ATCC NO. 16888), *Aspergillus terreus* (ATCC NO. 1012) and clinical isolate *Cryptococcus neoformans.* The nucleic acid sequences from phylogenetically near neighbours were also used as comparisons with the nucleic sequence from *Candida albicans* to determine variable regions.

### Example 1 ATCC Strains and Clinical Isolates

All the ten *Candida* species were purchased from American Type Culture Collection (ATCC). They were used as reference strains. Twenty-six clinical isolates of ten different *Candida* species were tested.

### Example 2 Growth Condition and DNA extraction

*Candida* cells were grown in 3 mL of Sabouraud Dextrose Broth (SDB) at 37°C overnight in a shaking incubator till they reached mid-log phase. Broth culture that contains the *Candida* cells were used for DNA extraction. Genomic DNA of the *Candida* cells were extracted using conventional phenol-chloroform extraction method. The cells were washed twice with 1 mL of phosphate buffered saline (PBS), pH 7.4 by centrifugation at 13,200 rpm for 2 minutes. The cell walls were broken down by adding 500 µL lysis buffer (1 M Tris-HCl, 0.5 M EDTA, 0.5% v/v β-mercaptoethanol) at 37°C in a shaking incubator. The cells were later lysed to release the DNA from the nucleus by adding 50 µL of 10% sodium dodecyl sulfate (SDS) and the cell debris were denatured using 2.5 µL 20 mg/mL proteinase K. Equal volume (approx. 553 µL) of phenol: chloroform: isoamyalcohol was added to separate the DNA from histones and protein. Released DNA were precipitated and concentrated by adding 1/10 volume (50 µL) of 3 M sodium acetate, pH 5.2 and equal volume of isopropanol (500 µL) and incubated for 10 minutes at room temperature. The DNA were pelleted through centrifugation at 13,200 rpm for 15 minutes, washed with 70% cold ethanol, spun at 13,200 rpm for 5 minutes and finally resuspended in sterile distilled water or TE buffer, pH 7.4 (10 mM Tris-Cl, pH 7.4, 1 mM EDTA, pH 8.0) for longer preservation.

### Example 3 Polymerase Chain Polymerase

Eppendorf Mastercycler® gradient PCR machine was used for the DNA amplification process. The reaction mixture for polymerase chain reaction (PCR) consists of PCR buffer, magnesium chloride (MgCl₂), deoxynucleotide triphosphates (dNTPs), forward primer and reverse primer and *Taq* DNA polymerase enzyme. All the PCR reaction ingredients are from Fermentas Life Sciences. The first round of PCR was carried out using the primers SEQ ID NO: 11 TCC GTA GGT GAA CCT GCG G and SEQ ID NO: 12 TCC TCC GCT TAT TGA TAT GC. Table 1 shows list of primers used in the first and second round of the semi-nested PCR process. The PCR reaction mixtures for the first round of PCR, using SEQ ID NO: 11 and SEQ ID NO: 12 as primers are shown in Table 2 whereas the PCR program is shown in Table 5, respectively. For the second round PCR depending on the species, the SEQ ID NO: 11 remains and ten different species specific reverse primers for the respective species (SEQ ID NO: 1, 2, 3, 4, 5, 6, 7, 8, 9, 10) were used accordingly. One microlitre of the first round PCR product was used as template for the second round PCR. The details of the PCR reaction mixture and PCR program for every species PCR reactions are shown in Table 3 and Table 5. All the PCR products obtained are subjected to agarose gel electrophoresis at 72 volts for 45 minutes and later viewed using the gel documentation system.

**Table 1**

| Species | Primer | Length (bp) | Sequence 5' to 3' | Region |
|---|---|---|---|---|
| All fungi | SEQ ID NO: 11 | 19 | TCC GTA GGT GAA CCT GCG G | 18S rRNA |
| All fungi | SEQ ID NO: 12 | 20 | TCC TCC GCT TAT TGA TAT GC | 28S rRNA |
| *C. albicans* | SEQ ID NO: 1 | 21 | CTA AGC CAT TGT CAA AGC GAT | ITS2 |
| *C. dubliniensis* | SEQ ID NO: 2 | 22 | CTA AGC CAT TGT CAA GCA TCT C | ITS2 |
| *C. glabrata* | SEQ ID NO: 3 | 20 | CAC TCC CAG GTC TTT GTC GG | ITS1 |
| *C. guilliermondii* | SEQ ID NO: 4 | 20 | GAA ATA TCC CGC CAC ACC AT | ITS2 |
| C. *kefyr* | SEQ ID NO: 5 | 24 | CGA GTC TTT ATA ACA CCT ATG AGT | ITS2 |
| *C*. *krusei* | SEQ ID NO: 6 | 19 | CAG CTT CGC TCC CTT TCA G | ITS2 |
| *C. lusitaniae* | SEQ ID NO: 7 | 19 | TTC GGA GCA ACG CCT AAC C | ITS2 |
| *C. parapsilosis* | SEQ ID NO: 8 | 23 | CCA ATG AGT GGA AAA AAC CTA TC | ITS2 |
| *C. rugosa* | SEQ ID NO: 9 | 22 | CTT AAC TGT TTT AGA CGG TCG C | ITS2 |
| *C. tropicalis* | SEQ ID NO: 10 | 23 | TTT TTG GAT AAA CCT AAG TCG CT | ITS2 |

**Table 2**

| **PCR component** | | **Initial Concentration** | **Final Concentration** | **Volume, µL** |
|---|---|---|---|---|
| DNA | | - | - | 1.0 |
| PCR Buffer | | 10 X | 1 X | 2.0 |
| MgCl₂ | | 25 mM | 2 mM | 1.6 |
| dNTPs | | 10 mM each | 0.25 mM each | 0.5 |
| Primer (SEQ ID NO: 11) | | 10 µM | 0.25 µM | 0.5 |
| | (SEQ ID NO: 12) | 10 µM | 0.25 µM | 0.5 |
| *Taq* DNA polymerase | | 1 U/µL | 0.5 U | 0.5 |
| ddH₂O | | - | - | 13.4 |
| Total volume: | | | | 20 |

**Table 3**

| **PCR component** | | **Initial Concentration** | **Final Concentration** | **Volume** |
|---|---|---|---|---|
| First round PCR product | | - | - | 1.0 |
| PCR Buffer | | 10 X | 1X | 2.0 |
| MgCl₂ | | 25 mM | (depending on species - Refer Table 4) | (depending on species - Refer Table 4) |
| dNTPs | | 10 mM each | 0.25 mM each | 0.5 |
| Primer (SEQ ID NO: 11) | | 10 µM | 0.25 µM | 0.5 |
| | (SEQ ID NO: X) | 10 µM | 0.25 µM | 0.5 |
| *Taq* DNA polymerase | | 1 U/µL | 0.5 U | 0.5 |
| ddH₂O | | - | - | (top up to 20 µl) |

| | | | | |
|---|---|---|---|---|
| # 'X' can be SEQ ID NO: 1 or SEQ ID NO: 2 or SEQ ID NO: 3 or SEQ ID NO: 4 or SEQ ID NO: 5 or SEQ ID NO: 6 or SEQ ID NO: 7 or SEQ ID NO: 8 or SEQ ID NO: 9 or SEQ ID NO: 10. | | | | |

**Table 4**

| **Species** | **Final volume, µL** | **MgCl₂ concentration, mM** |
|---|---|---|
| *C. albicans* | 1.2 | 1.50 |
| *C. dubliniensis* | 1.2 | 1.50 |
| *C. glabrata* | 1.2 | 1.50 |
| *C. guilliermondii* | 1.2 | 1.50 |
| C. *kefyr* | 1.2 | 1.50 |
| *C. krusei* | 1.6 | 2.00 |
| *G lusitaniae* | 1.4 | 1.75 |
| *C. parapsilosis* | 1.2 | 1.50 |
| *C. rugosa* | 1.6 | 2.00 |
| *C. tropicalis* | 1.2 | 1.50 |
| *A. flavus* | 1.2 | 1.50 |
| *A. fumigatus* | 1.1 | 1.375 |
| *A. nidulans* | 1.0 | 1.25 |
| *A. niger* | 1.0 | 1.25 |
| *A. terreus* | 1.0 | 1.25 |
| *C. neoformans* | 1.2 | 1.50 |

**Table 5**

| **Step/Time Species** | **Initial Denaturation** | **Denaturation** | **Annealing** | **Extension** | **Final extension** |
|---|---|---|---|---|---|
| All fungi | 95°C, 2 mins | 95°C, 30 secs | 58.0°C, 30 secs | 72°C, 20 secs | 72°C, 4 mins |
| *C. albicans* | 95°C. 2 mins | 95°C, 30 secs | 60.0°C, 30 secs | 72°C, 20 secs | 72°C, 4 mins |
| *C. dubliniensis* | 95°C. 2 mins | 95°C. 30 secs | 60.0°C, 30 secs | 72°C, 20 secs | 72°C, 4 mins |
| *C. glabrata* | 95°C. 2 mins | 95°C, 30 secs | 65.1°C. 30 secs | 72°C, 30 secs | 72°C, 4 mins |
| *C. guilliermondii* | 95°C. 2 mins | 95°C. 30 secs | 60.0°C. 30 secs | 72°C, 30 secs | 72°C, 4 mins |
| *C. kefyr* | 95°C, 2 mins | 95°C, 30 secs | 65.0°C, 30 secs | 72°C, 20 secs | 72°C, 4 mins |
| C. *krusei* | 95°C, 2 mins | 95°C. 30 secs | 55.0°C, 30 secs | 72°C, 30 secs | 72°C, 4 mins |
| *C. lusitaniae* | 95°C, 2 mins | 95°C, 30 secs | 66.3°C, 30 secs | 72°C, 20 secs | 72°C, 4 mins |
| *C. parapsilosis* | 95°C, 2 mins | 95°C, 30 secs | 58.0°C, 30 secs | 72°C, 30 secs | 72°C, 4 mins |
| *C. rugosa* | 95°C, 2 mins | 95°C, 30 secs | 55.0°C, 30 secs | 72°C, 30 secs | 72°C, 4 mins |
| *C. tropicalis* | 95°C, 2 mins | 95°C, 30 secs | 60.0°C, 30 secs | 72°C, 20 secs | 72°C, 4 mins |

| | | | | | |
|---|---|---|---|---|---|
| • The PCR cycle repeats for 34 cycles, one cycle is defined as one round of denaturation, annealing and extension step. | | | | | |

### Example 4 Specificity and Sensitivity

Specificity testing was determined by using DNA of the ten *Candida* species (ATCC strains), six *Aspergillus* species (ATCC strains) and *Cryptococcus neoformans* (non-ATCC strain). The DNA amount template for the PCR is standardised at 150ng/µL. Sensitivity testing was determined by performing the PCR using a factor of ten times serial diluted DNA extracted from one million cells/conidia.

At the optimized semi-nested species specific PCR amplification condition, no amplicons were observed for all the species tested namely the ten *Candida* species (*C*. *albicans, C. dubliniensis, C. glabrata, C. guilliermondii, C. kefyr, C. krusei, C. lusitaniae, C. parapsilosis, C. rugosa* and *C*. *tropicalis),* six *Aspergillus* species *(A. flavus, A. fumigatus, A. lentulus, A. nidulans, A. niger, A. terreus), Cryptococcus neoformans* and also human DNA other than the targeted ones. Twenty-six *Candida* clinical isolates were also tested and they were identified accurately according to the species. All these results implied that the species specific semi-nested PCR is able to differentiate and identify the correct species among the commonly found medically important fungi and ruled out the possibility of amplifying human DNA and avoid false positive results. Furthermore, it can be elaborated that there were no cross-amplification of the species specific primers towards other fungi and human DNA other than the one tested and thus confirming the specificity of the semi-nested for all the ten *Candida* species.

DNA of one million cells of the ten medically important *Candida* cells that were extracted using the phenol: chloroform method was subjected to sensitivity testing. It was determined by performing the PCR using a factor of ten times serial diluted DNA extracted from one million cells/conidia from every species. It was found out that for this testing, PCR amplicons were seen from as low as one cell (*Candida parapsilosis* and *Candida rugosa),* ten cells (*Candida albicans, Candida dubliniensis, Candida glabrata, Candida krusei, Candida lusitaniae* and *Candida tropicalis)* and hundred cells (*Candida guilliermondii* and *Candida kefyr*). These results are shown in Table 6 as well as Figure 2 to 11.

The data shown in the examples described above confirm that the novel amplification oligonucleotides herein described and claimed are capable of amplifying the ten medically important *Candida* species nucleic acid and can be used in an assay to distinguish among themselves and from each other, the closest known phylogenetic neighbours. None of the examples described herein are intended to limit the present invention to the embodiments of this disclosure, said invention being limited exclusively by the claims which follow.

**Table 6**

| **Species** | | **Sensitivity** |
|---|---|---|
| | **Cells** | **Equivalent DNA amount** |
| *C. albicans* | 10¹ | 430 fg |
| *C. dubliniensis* | 10¹ | 400 fg |
| *C. glabrata* | 10¹ | 280 fg |
| *C. guilliermondii* | 10² | 2.6 pg |
| *C. kefyr* | 10² | 3.9 pg |
| *C. krusei* | 10¹ | 370 fg |
| *C. lusitaniae* | 10¹ | 410 fg |
| *C. parapsilosis* | 10⁰ | 44 fg |
| *C. rugosa* | 10⁰ | 46 fg |
| *C. tropicalis* | 10¹ | 250 fg |

## Claims

1. A method for detecting and identifying *Candida* species comprising the step of amplifying a conserved DNA segment of *Candida* species in a biological sample using a primer set having a nucleotide sequence of SEQ ID NO: 11 and a nucleotide sequence of SEQ ID NO: 12 in a first polymerase chain reaction; and conducting a second polymerase chain reaction by applying the amplified conserved DNA segment as a template and using a primer set having a nucleotide sequence of SEQ ID NO: 11 and a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 or SEQ ID NO: 10.

2. A method for species-specifically detecting and identifying *Candida albicans* by applying the amplified conserved DNA segment according to claim 1 as a template and using a primer set having a nucleotide sequence of SEQ ID NO: 11 and a nucleotide sequence of SEQ ID NO: 1 or the complementary sequence thereof in a second polymerase chain reaction.

3. A method for species-specifically detecting and identifying *Candida dubliniensis* by applying the amplified conserved DNA segment according to claim 1 as a template and using a primer set having a nucleotide sequence of SEQ ID NO: 11 and a nucleotide sequence of SEQ ID NO: 2 or the complementary sequence thereof in a second polymerase chain reaction.

4. A method for species-specifically detecting and identifying *Candida glabrata* by applying the amplified conserved DNA segment according to claim 1 as a template and using a primer set having a nucleotide sequence of SEQ ID NO: 11 and a nucleotide sequence of SEQ ID NO: 3 or the complementary sequence thereof in a second polymerase chain reaction.

5. A method for species-specifically detecting and identifying *Candida guilliermondii* by applying the amplified conserved DNA segment according to claim 1 as a template and using a primer set having a nucleotide sequence of SEQ ID NO: 11 and a nucleotide sequence of SEQ ID NO: 4 or the complementary sequence thereof in a second polymerase chain reaction.

6. A method for species-specifically detecting and identifying *Candida kefyr* by applying the amplified conserved DNA segment according to claim 1 as a template and using a primer set having a nucleotide sequence of SEQ ID NO: 11 and a nucleotide sequence of SEQ ID NO: 5 or the complementary sequence thereof in a second polymerase chain reaction.

7. A method for species-specifically detecting and identifying *Candida krusei* by applying the amplified conserved DNA segment according to claim 1 as a template and using a primer set having a nucleotide sequence of SEQ ID NO: 11 and a nucleotide sequence of SEQ ID NO: 6 or the complementary sequence thereof in a second polymerase chain reaction.

8. A method for species-specifically detecting and identifying *Candida lusitaniae* by applying the amplified conserved DNA segment according to claim 1 as a template and using a primer set having a nucleotide sequence of SEQ ID NO: 11 and a nucleotide sequence of SEQ ID NO: 7 or the complementary sequence thereof in a second polymerase chain reaction.

9. A method for species-specifically detecting and identifying *Candida parapsilosis* by applying the amplified conserved DNA segment according to claim 1 as a template and using a primer set having a nucleotide sequence of SEQ ID NO: 11 and a nucleotide sequence of SEQ ID NO: 8 or the complementary sequence thereof in a second polymerase chain reaction.

10. A method for species-specifically detecting and identifying *Candida rugosa* by applying the amplified conserved DNA segment according to claim 1 as a template and using a primer set having a nucleotide sequence of SEQ ID NO: 11 and a nucleotide sequence of SEQ ID NO: 9 or the complementary sequence thereof in a second polymerase chain reaction.

11. A method for species-specifically detecting and identifying *Candida tropicalis* by applying the amplified conserved DNA segment according to claim 1 as a template and using a primer set having a nucleotide sequence of SEQ ID NO: 11 and a nucleotide sequence of SEQ ID NO: 10 or the complementary sequence thereof in a second polymerase chain reaction.

12. A polymerase chain reaction (PCR)-based amplification kit for detecting and identifying *Candida* species in a biological sample by a method according to any of the preceding claims, **characterized in that** the kit comprises a primer set having a nucleotide sequence of SEQ ID NO: 11 and a nucleotide sequence of SEQ ID NO: 12, and a primer set having a nucleotide sequence of SEQ ID NO: 11 and a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 and SEQ ID NO: 10.

## Patentansprüche

1. Verfahren zum Nachweisen und Identifizieren von *Can*dida-Spezies, umfassend den Schritt der Amplifizierung eines konservierten DNA-Segments einer *Candida-*Spezies in einer biologischen Probe unter Verwendung eines Primersatzes, der eine Nukleotidsequenz von SEQ-ID-NO: 11 und eine Nukleotidsequenz von SEQ-ID-NO: 12 aufweist, in einer ersten Polymerasekettenreaktion; und Durchführung einer zweiten Polymerasekettenreaktion durch Einsatz des amplifizierten konservierten DNA-Segments als Matrize und Verwendung eines Primersatzes, der eine Nukleotidsequenz von SEQ-ID-NO: 11 und eine Nukleotidsequenz, ausgewählt aus der Gruppe, die aus SEQ-ID-NO: 1, SEQ-ID-NO: 2, SEQ-ID-NO: 3, SEQ-ID-NO: 4, SEQ-ID-NO: 5, SEQ-ID-NO: 6, SEQ-ID-NO: 7, SEQ-ID-NO: 8, SEQ-ID-NO: 9 und SEQ-ID-NO: 10 besteht, aufweist.

2. Verfahren zum spezies-spezifischen Nachweisen und Identifizieren von *Candida albicans* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 1 oder die komplementäre Sequenz davon aufweist, in einer zweiten Polymerasekettenreaktion.

3. Verfahren zum spezies-spezifischen Nachweisen und Identifizieren von *Candida dubliniensis* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 2 oder die komplementäre Sequenz davon aufweist, in einer zweiten Polymerasekettenreaktion.

4. Verfahren zum spezies-spezifischen Nachweisen und Identifizieren von *Candida glabrata* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 3 oder die komplementäre Sequenz davon aufweist, in einer zweiten Polymerasekettenreaktion.

5. Verfahren zum spezies-spezifischen Nachweisen und Identifizieren von *Candida guilliermondii* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 4 oder die komplementäre Sequenz davon aufweist, in einer zweiten Polymerasekettenreaktion.

6. Verfahren zum spezies-spezifischen Nachweisen und Identifizieren von *Candida kefyr* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 5 oder die komplementäre Sequenz davon aufweist, in einer zweiten Polymerasekettenreaktion.

7. Verfahren zum spezies-spezifischen Nachweisen und Identifizieren von *Candida krusei* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 6 oder die komplementäre Sequenz davon aufweist, in einer zweiten Polymerasekettenreaktion.

8. Verfahren zum spezies-spezifischen Nachweisen und Identifizieren von *Candida lusitaniae* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 7 oder die komplementäre Sequenz davon aufweist, in einer zweiten Polymerasekettenreaktion.

9. Verfahren zum spezies-spezifischen Nachweisen und Identifizieren von *Candida parapsilosis* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 8 oder die komplementäre Sequenz davon aufweist, in einer zweiten Polymerasekettenreaktion.

10. Verfahren zum spezies-spezifischen Nachweisen und Identifizieren von *Candida rugosa* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 9 oder die komplementäre Sequenz davon aufweist, in einer zweiten Polymerasekettenreaktion.

11. Verfahren zum spezies-spezifischen Nachweisen und Identifizieren von *Candida tropicalis* durch Einsatz des amplifizierten konservierten DNA-Segments nach Anspruch 1 als Matrize und Verwendung eines Primersatzes, der eine Nukleotidsequenz von SEQ-ID-NO: 11 und eine Nukleotidsequenz von SEQ-ID-NO: 10 oder die komplementäre Sequenz davon aufweist, in einer zweiten Polymerasekettenreaktion.

12. Polymerasekettenreaktions (PCR)-basierter Amplifizierungskit zum Nachweisen und Identifizieren von *Candida-*Spezies in einer biologischen Probe mit einem Verfahren nach einem der vorhergehenden Ansprüche, **dadurch gekennzeichnet, dass** der Kit umfasst einen Primersatz, der eine Nukleotidsequenz von SEQ-ID-NO: 11 und eine Nukleotidsequenz von SEQ-ID-NO: 12 aufweist, und einen Primersatz, der eine Nukleotidsequenz von SEQ-ID-NO: 11 und eine Nukleotidsequenz, ausgewählt aus der Gruppe, die aus SEQ-ID-NO: 1, SEQ-ID-NO: 2, SEQ-ID-NO: 3, SEQ-ID-NO: 4, SEQ-ID-NO: 5, SEQ-ID-NO: 6, SEQ-ID-NO: 7, SEQ-ID-NO: 8, SEQ-ID-NO: 9 und SEQ-ID-NO: 10 besteht, aufweist.

## Revendications

1. Procédé de détection et d'identification d'espèces de *Candida* comprenant l'étape d'amplification d'un segment d'ADN conservé d'espèces de *Candida* dans un échantillon biologique en utilisant un jeu d'amorces ayant une séquence nucléotidique de SEQ ID N° : 11 et une séquence nucléotidique de SEQ ID N° : 12 dans une première réaction en chaîne par polymérase ; et la réalisation d'une deuxième réaction en chaîne par polymérase en appliquant le segment d'ADN conservé amplifié comme matrice et en utilisant un jeu d'amorces ayant une séquence nucléotidique de SEQ ID N° : 11 et une séquence nucléotidique choisie dans le groupe consistant en SEQ ID N° : 1, SEQ ID N° : 2, SEQ ID N° : 3, SEQ ID N° : 4, SEQ ID N° : 5, SEQ ID N° : 6, SEQ ID N° : 7, SEQ ID N° : 8, SEQ ID N° : 9 ou SEQ ID N° : 10.

2. Procédé de détection spécifique d'une espèce et d'identification de *Candida albicans* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 1 ou la séquence complémentaire de celle-ci dans une deuxième réaction en chaîne par polymérase.

3. Procédé de détection spécifique d'une espèce et d'identification de *Candida dubliniensis* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 2 ou la séquence complémentaire de celle-ci dans une deuxième réaction en chaîne par polymérase.

4. Procédé de détection spécifique d'une espèce et d'identification de *Candida glabrata* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 3 ou la séquence complémentaire de celle-ci dans une deuxième réaction en chaîne par polymérase.

5. Procédé de détection spécifique d'une espèce et d'identification de *Candida guilliermondii* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 4 ou la séquence complémentaire de celle-ci dans une deuxième réaction en chaîne par polymérase.

6. Procédé de détection spécifique d'une espèce et d'identification de *Candida kefyr* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 5 ou la séquence complémentaire de celle-ci dans une deuxième réaction en chaîne par polymérase.

7. Procédé de détection spécifique d'une espèce et d'identification de *Candida krusei* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 6 ou la séquence complémentaire de celle-ci dans une deuxième réaction en chaîne par polymérase.

8. Procédé de détection spécifique d'une espèce et d'identification de *Candida lusitaniae* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 7 ou la séquence complémentaire de celle-ci dans une deuxième réaction en chaîne par polymérase.

9. Procédé de détection spécifique d'une espèce et d'identification de *Candida parapsilosis* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 8 ou la séquence complémentaire de celle-ci dans une deuxième réaction en chaîne par polymérase.

10. Procédé de détection spécifique d'une espèce et d'identification de *Candida rugosa* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 9 ou la séquence complémentaire de celle-ci dans une deuxième réaction en chaîne par polymérase.

11. Procédé de détection spécifique d'une espèce et d'identification de *Candida tropicalis* en appliquant le segment d'ADN conservé amplifié selon la revendication 1 comme matrice et en utilisant un jeu d'amorces ayant une séquence nucléotidique de SEQ ID N° : 11 et une séquence nucléotidique de SEQ ID N° : 10 ou la séquence complémentaire de celle-ci dans une deuxième réaction en chaîne par polymérase.

12. Kit d'amplification basé sur une réaction en chaîne par polymérase (PCR) pour détecter et identifier des espèces de *Candida* dans un échantillon biologique par un procédé selon l'une quelconque des revendications précédentes, **caractérisé en ce que** le kit comprend un jeu d'amorces ayant une séquence nucléotidique de SEQ ID N° : 11 et une séquence nucléotidique de SEQ ID N° 12, et un jeu d'amorces ayant une séquence nucléotidique de SEQ ID N° : 11 et une séquence nucléotidique choisie dans le groupe consistant en SEQ ID N° : 1, SEQ ID N° : 2, SEQ ID N° : 3, SEQ ID N° : 4, SEQ ID N° : 5, SEQ ID N° : 6, SEQ ID N° : 7, SEQ ID N° : 8, SEQ ID N° : 9 et SEQ ID N° : 10.
